Europäisches Patentamt

European Patent Office

Office européen des brevets

(11) Publication number: **0 653 400 A1**

# EUROPEAN PATENT APPLICATION

(21) Application number: 94203291.3

(22) Date of filing: 11.11.94

(51) Int. Cl.⁶: **C07C 9/00**, C07C 5/27

(30) Priority: 12.11.93 EP 93402761

(43) Date of publication of application:
17.05.95 Bulletin 95/20

(84) Designated Contracting States:
DE DK FR GB NL SE

(71) Applicant: SHELL INTERNATIONALE
RESEARCH MAATSCHAPPIJ B.V.
Carel van Bylandtlaan 30
NL-2596 HR Den Haag (NL)

(72) Inventor: Grandvallet, Pierre
Badhuisweg 3
NL-1031 CM Amsterdam (NL)
Inventor: De Jong, Krijn Peter
Badhuisweg 3
NL-1031 CM Amsterdam (NL)
Inventor: Reinalda, Donald
Badhuisweg 3
NL-1031 CM Amsterdam (NL)

(54) Process for the preparation of multi-branched paraffins.

(57) Process for the preparation of multi-branched paraffins comprising contacting a hydrocarbonaceous feedstock substantially boiling in the gasoline range which feedstock comprises linear paraffins having at least five carbon atoms in a first reaction stage in the presence of hydrogen and at elevated temperature and pressure with a first isomerisation catalyst which comprises a molecular sieve comprising pores with a diameter D1 and at least one Group VIII metal component to produce mono-branched paraffins, and subsequently contacting at least part of the effluent so obtained in a second reaction stage at elevated temperature and pressure and in the presence of hydrogen with a second isomerisation catalyst comprising pores with a diameter D2 and at least one Group VIII metal component to produce multi-branched paraffins, whereby D1/D2 is at most 1.

The present invention relates to a process for the preparation of multi-branched paraffins from a hydrocarbonaceous feedstock substantially boiling in the gasoline range which comprises linear paraffins having at least five carbon atoms.

One of the main objects in nowaday's oil refining is to produce gasolines fulfilling the increasing environmental demands on product quality and having a high octane number.

This means for gasoline that the octane specification has now to be established for instance without lead-containing additives, less aromatics, in particular benzene, and less olefins.

Components which contribute to the octane quality of gasoline include branched paraffins, in particular multi-branched paraffins. Object of the present invention is to provide multi-branched paraffins, in particular multi-branched $C_7$ paraffins which are very attractive high octane gasoline components.

It has now been found that remarkably high yields of multi-branched paraffins, in particular multi-branched $C_7$ paraffins predominantly existing of double-branched $C_7$ paraffins, can be obtained from a hydrocarbonaceous feedstock substantially boiling in the gasoline range which feedstock comprises linear paraffins having at least five carbon atoms when use is made of a specific two-stage isomerisation process.

Accordingly, the present invention relates to a process for the preparation of multi-branched paraffins comprising contacting a hydrocarbonaceous feedstock substantially boiling in the gasoline range which feedstock comprises linear paraffins having at least five carbon atoms in a first reaction stage in the presence of hydrogen and at elevated temperature and pressure with a first isomerisation catalyst which comprises a molecular sieve comprising pores with a diameter D1 and at least one Group VIII metal component to produce mono-branched paraffins, and subsequently contacting at least part of the effluent so obtained in a second reaction stage at elevated temperature and pressure and in the presence of hydrogen with a second isomerisation catalyst comprising pores with a diameter D2 and at least one Group VIII metal component to produce multi-branched paraffins, whereby D1/D2 is at most 1.

In the context of the present invention diameter D1 is defined as the largest average pore diameter present in the molecular sieve crystallites composing the catalyst, whereas diameter D2 is defined as the largest average pore diameter present in the component of the second isomerisation catalyst bearing the acidic function (e.g. zeolite or amorphous silica-alumina).

In case the molecular sieve comprises pores of an elliptical cross-section having a minor axis (A) and a major axis (B), D1 is defined as (A + B)/2.

Diameters D1 and D2 can be determined by means of or derived from techniques well known in the art. In case the second isomerisation catalyst has an amorphous structure, D2 can for instance be determined by means of Mercury Intrusion Porosimetry.

Suitably, the first stage isomerisation catalyst comprises a medium-pore molecular sieve comprising pores with a diameter D1 in the range of 3.8 to 7 Å, preferably in the range of 4 to 6.5 Å.

Suitably, the first stage isomerisation catalyst comprises a molecular sieve selected from the AEL family of zeolites as described in Zeolites, The International Journal of Molecular Sieves, Vol. 12, No. 8, June 1992. Suitably, the pores with a diameter D1 are ten ring pores.

Suitably, the molecular sieve of the first stage isomerisation catalyst comprises a MeAPO and/or MeAPSO medium-pore molecular sieve, wherein Me is at least Mg, Mn, Co or Zn.

Preferably, a combination of catalysts is applied wherein D1/D2 is less than 1.

In the context of the present invention a MeAPO medium-pore molecular sieve is defined as a crystalline microporous metal aluminophosphate composition having a three-dimensional framework structure of interconnected $MeO_2$, $AlO_2$ and $PO_2$ tetrahedral units. This type of molecular sieve and its preparation have for instance been described in US 4,567,029 which is hereby incorporated by reference.

Moreover, in the context of the present invention a MeAPSO medium-pore molecular sieve is defined as a crystalline microporous metal silicoaluminophosphate having a three-dimensional framework structure of interconnected $MeO_2$, $AlO_2$ $SiO_2$ and $PO_2$ tetrahedral units. MeAPSO medium-pore molecular sieves and their preparation have for example been disclosed in EP 158348, EP 158975, EP 161489 and EP 161490 which are hereby incorporated by reference.

Preferably, the first stage isomerisation catalyst comprises a MeAPO and/or MeAPSO medium-pore molecular sieve, wherein Me is at least Co.

Suitable CoAPO and CoAPSO catalysts include CoAPO-11 and CoAPSO-11 catalysts.

Suitably, use is made of a MeAPO and/or MeAPSO medium-pore molecular sieve having an anhydrous composition which can be expressed in molar oxide ratios as follows: $(MeO)_a(Al_2O_3)_b$-$(P_2O_5)_c(SiO_2)_d$, wherein a ranges from 0.003 to 0.2, preferably from 0.005 to 0.15, b ranges from 0.05 to 0.3, preferably from 0.05 to 0.27, c ranges from 0.05 to 0.3, preferably from 0.05 to 0.27, and d is at most 0.4, preferably at most 0.3.

In another attractive embodiment of the present invention the first isomerisation catalyst comprises a ferrierite catalyst (with ten ring pores) comprising at least one Group VIII metal component.

Suitably, the ferrierite catalyst to be applied has a $SiO_2/Al_2O_3$ molar ratio of up to 120, preferably from 15 to 100.

Preferably, the ferrierite catalyst is substantially in its hydrogen form.

It should be noted that in the context of the present invention the term ferrierite catalyst includes apart from ferrierite as such other tectometallosilicates having a ferrierite structure. Such tectometallosilicates include FU-9, ISI-6, Nu-23, ZSM-21, ZSM-35 and ZSM-38. However, ferrierite as such is preferred.

Suitably, the pores of the second stage isomerisation catalyst with diameter D2 are twelve ring, or even larger pores.

Suitably, D2 is in the range of 7 to 1000 Å, preferably in the range of 7 to 100 Å.

Suitably, the second stage isomerisation catalyst comprises a specific heterogeneous hydroisomerisation catalyst having acid activity and a hydrogenation activity. The catalyst comprises one or more metals from Group VIII of the Periodic Table of the Elements on a carrier material. The carrier material has acidic properties and may suitably consist of amorphous mixed oxides, in particular amorphous silica-alumina, and zeolites, in particular mordenite, faujasite in the hydrogen form or exchanged with rare-earth ions, or of alumina rendered acidic by combination with halogen (e.g. chlorine). Preferably, the second isomerisation catalyst comprises at least one noble-metal from Group VIII (in particular platinum and/or palladium) on H-mordenite as carrier material. Most preferably, the H-mordenite is prepared by treating mordenite one or more times with an aqueous solution of an acid (e.g. hydrochloric acid) and, separately, one or more times with an aqueous solution of an ammonium compound (e.e. ammonium nitrate), followed by drying (e.g. at 100-200 °C) and calcining (e.g. at 400-700 °C) of the treated mordenite. The (noble) metal(s) may be incorporated into the carrier material by any method known in the art, such as impregnation, precipitation or, preferably ion-exchange. If desired, the carrier material can be mixed with an inert binder before or after incorporation of catalytically active metal(s). Suitable binders are e.g. natural clays (such as kaolin or bentonite) and refractory oxides such as alumina, silica, boria, chromia and zirconia or combinations thereof.

The first and second isomerisation catalysts comprise at least one Group VIII metal component. Suitably, the Group VIII metal component comprises a noble-metal-component, preferably a platinum and/or palladium component, and more preferably a platinum and palladium component. Suitably, the catalyst comprises 0.01-5% by weight of Group VIII metal based on total catalyst. Preferably, the catalyst comprises 0.1 to 3% by weight of Group VIII metal based on total catalyst. Preferably, a substantial amount of the Group VIII metal component is in metallic form which can be achieved by reduction procedures known in the art. More preferably, the entire amount of the Group VIII metal component is in metallic form. The catalyst may further comprise a binder material comprising one or more refractory oxides. The Group VIII metal component may be present on the medium-pore molecular sieve structure or may be present on a separate carrier (e.g. the binder) comprising, for instance, one or more refractory oxides, e.g. alumina. Preferably, the Group VIII metal component is present on the molecular sieve structure. Suitably, the Group VIII metal component is loaded on the molecular sieve structure by means of ion-exchange or impregnation, although other methods known in the art may be applied. Suitably, the Group VIII metal component is distributed in the molecular sieve structure, though this may not be achieved by conventional loading techniques. A possible route for incorporation of the Group VIII metal is to add it to the molecular sieve synthesis mixture.

The hydrocarbonaceous feedstock substantially boiling in the gasoline range can suitably be obtained by fractionating any paraffinic gasoline-containing hydrocarbonaceous feedstock. Preferably the feedstock substantially boiling in the gasoline range is obtained by subjecting a straight run hydrocarbon oil to a fractionation, preferably atmospheric distillation. Other suitable hydrocarbonaceous feedstocks substantially boiling in the gasoline range include product fractions obtained from cracking processes such as catalytic cracking, thermal cracking, delayed coking, visbreaking and flexicoking. Hydrocarbonaceous feedstocks containing unacceptable levels of sulphur and nitrogen may be subjected to a hydrotreatment before they are subjected to the process according to the present invention, whereby particularly advantageous results may be obtained. Suitably the hydrocarbonaceous feedstock boiling in the gasoline range has a low sulphur content, preferably less than 10 ppmw on feed.

The hydrocarbonaceous feedstock may consist entirely of a fraction boiling in the gasoline range, i.e. in the range of $C_4$ -220 °C. While the full gasoline boiling range fraction may be included in the hydrocarbonaceous feedstock, it may be preferred to employ as hydrocarbonaceous feedstock a cut thereof having a boiling point up to 180 °C. Optionally, the hydrocarbonaceous feedstock may be blended with a reformate fraction.

Suitably, the hydrocarbonaceous feedstock substantially boiling in the gasoline range may substantially comprise linear paraffins having at least five carbon atoms, i.e. the feedstock may substantially consist of one or more different types of linear paraffins having at least five carbon atoms.

Preferably, the hydrocarbonaceous feedstock boiling in the gasoline range substantially comprises linear paraffins having five to ten carbon atoms. More preferably, the hydrocarbonaceous feedstock substantially comprises linear paraffins having six to eight carbon atoms. Suitably, the hydrocarbonaceous feedstock substantially comprises n-heptane. Apart from n-heptane the feedstock may contain $C_7$ naphthenes and mono-branched $C_7$ paraffins.

In accordance with the present invention the first and second reaction stage can be carried out in separate vessels, or they may be carried out in a stacked-bed flow scheme within one reactor.

When use is made of a ferrierite catalyst comprising ten ring pores the process is suitably carried out in the first reaction stage at a temperature of 250 to 350 °C, a pressure of up to 50 bar, a space velocity of 0.5 to 10 g/g/h and a $H_2$/feedstock molar ratio of less than 5. Preferably, the process is carried out in the first reaction stage with a ferrierite catalyst at a temperature of 275 to 325 °C, a pressure of 10 to 30 bar, a space velocity of 1 to 5 g/g/h and a $H_2$/feedstock molar ratio of from 1 to 3.

When use is made of a MeAPO and/or MeAPSO medium-pore molecular sieve the process is suitably carried out in the first stage at a temperature of 250 to 450 °C, a pressure of up to 100 bar, a space velocity of 0.5 to 10 g/g/h and a $H_2$/feedstock molar ratio of less than 5. Preferably, the process is carried out in the first reaction stage with a MeAPO and/or MeAPSO medium-pore molecular sieve at a temperature of 275 to 400 °C, a pressure of 10 to 50 bar, a space velocity of 1 to 5 g/g/h and a $H_2$/feedstock molar ratio of 1-3.

Suitably, the process is carried out in the second stage at a temperature of 50 to 400 °C, a pressure of up to 50 bar, a space velocity of 0.5 to 10 g/g/h and a $H_2$/feedstock molar ratio of less than 5.

When use is made in the second stage of an amorphous alumina-silica catalyst, the process is preferably carried out in the second reaction stage at a temperature of greater than 250 °C.

When use is made in the second stage of a mordenite catalyst, the process is preferably carried out in the second stage at a temperature of 180-280 °C. When use is made in the second stage of a $Pt/Cl/Al_2O_3$ catalyst, the process is preferably carried out in the second stage at a temperature of 50-150 °C.

It is especially the use of the particular isomerisation catalyst in each stage which allows the attractively high yield of double-branched paraffins, in particular double-branched $C_7$ paraffins.

Suitably, the total effluent from the first reaction stage is passed to the second reaction stage.

Suitably, unconverted linear paraffins are separated from the effluent upstream the second reaction zone, and recycled to the first reaction zone. In another embodiment of the present invention unconverted linear paraffins are separated from the effluent downstream the second reaction stage, and recycled to the first reaction stage. Unconverted mono-branched paraffins can be separated from the effluent downstream the second reaction stage, and recycled to the second reaction stage. Such a separation can, for instance, be carried out by means of a molecular sieve or by means of distillation.

The process in accordance with the present invention can be carried out in a number of alternative ways, and some process schemes according to the present invention will be elucidated more fully hereinafter, with reference to the accompanying Figures.

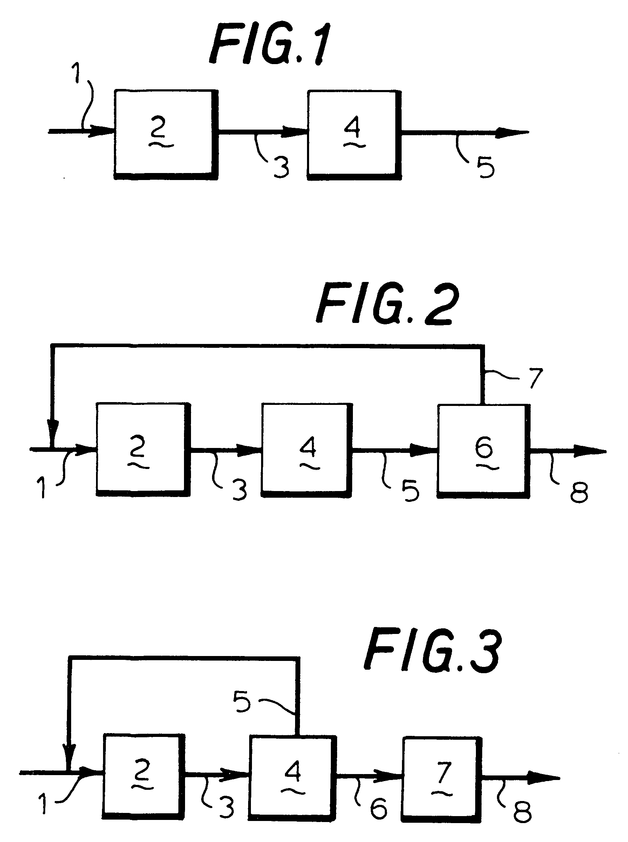

In the process schematically shown in Figure 1 a hydrocarbonaceous feedstock comprising n-heptane is passed via a line 1 to a first isomerisation unit 2. The effluent obtained from the first isomerisation unit 2 is passed via a line 3 to a second isomerisation unit 4. The effluent obtained from the second isomerisation 4 is subsequently recovered via a line 5.

The process schematically shown in Figure 2 differs from the process schematically shown in Figure 1 in that the effluent from the second isomerisation unit is passed via the line 5 to a separation unit 6. Separation unit 6 comprises a molecular sieve, with the help of which n-heptane is separated from cyclic and branched $C_7$ paraffins, thereby producing a stream comprising n-heptane which is recycled to the first isomerisation unit 2 via a line 7 and a stream which comprises cyclic and branched $C_7$ paraffins which is recovered via a line 8.

In another embodiment the process is carried out as shown in Figure 2, except that in addition in the separation unit 6 mono-branched $C_7$ paraffins are separated from cyclic and multi-branched $C_7$ paraffins, thereby producing a stream comprising mono-branched $C_7$ paraffins which is recycled to the second isomerisation unit 4 and a stream which comprises cyclic and multi-branched $C_7$ paraffins which is recovered via the line 8.

In yet another embodiment the process is carried out as depicted in Figure 2, except that the recycle stream (line 7) comprises both n-heptane and mono-branched $C_7$ paraffins, whereas the

stream which is recovered via the line 8 comprises cyclic and multi-branched $C_7$ paraffins, and both the isomerisation units 2 and 4 are arranged in a stacked-bed flow scheme within one reactor.

In the process schematically shown in Figure 3 the effluent from the first isomerisation unit 2 is passed via the line 3 to a separation unit 4. The separation unit 4 comprises a molecular sieve, with the help of which n-heptane is separated from cyclic and branched $C_7$ paraffins, thereby producing a stream comprising n-heptane which is recycled to the first isomerisation unit 2 via a line 5 and a stream which comprises cyclic and branched $C_7$ hydrocarbons which is passed via a line 6 to a second isomerisation unit 7. The effluent obtained from the second isomerisation unit 7 is recovered via a line 8.

**Claims**

1. Process for the preparation of multi-branched paraffins comprising contacting a hydrocarbonaceous feedstock substantially boiling in the gasoline range which feedstock comprises linear paraffins having at least five carbon atoms in a first reaction stage in the presence of hydrogen and at elevated temperature and pressure with a first isomerisation catalyst which comprises a molecular sieve comprising pores with a diameter D1 and at least one Group VIII metal component to produce mono-branched paraffins, and subsequently contacting at least part of the effluent so obtained in a second reaction stage at elevated temperature and pressure and in the presence of hydrogen with a second isomerisation catalyst comprising pores with a diameter D2 and at least one Group VIII metal component to produce multi-branched paraffins, whereby D1/D2 is at most 1.

2. Process according to claim 1, wherein D1/D2 is less than 1.

3. Process according to claim 1 or 2, wherein the first isomerisation catalyst comprises a medium-pore molecular sieve comprising pores with a diameter D1 in the range of 3.8 to 7 Å.

4. Process according to claim 3, wherein the first isomerisation catalyst comprises a ferrierite catalyst.

5. Process according to claim 3, wherein the first isomerisation catalyst comprises a MeAPO and/or MeAPSO molecular sieve, wherein Me is at least Mg, Mn, Co or Zn.

6. Process according to claim 5, wherein Me is at least Co.

7. Process according to any one of claims 1-6, wherein the second isomerisation catalyst comprises twelve ring pores.

8. Process according to any one of claims 1-7, wherein the catalysts comprise 0.01-5% by weight of the Group VIII metal based on total catalyst.

9. Process according to any one of claims 1-8, wherein the Group VIII metal component metal comprises a noble-metal component.

10. Process according to claim 9, wherein the Group VIII metal component comprises a Pt and/or Pd component.

11. Process according to any one of claims 1-10, wherein the second isomerisation catalyst comprises a mordenite catalyst.

12. Process according to any one of claims 4-11, which is carried out in the first stage at a temperature of 250 to 350 °C, a pressure of up to 50 bar, a space velocity of 0.5 to 10 g/g/h and a $H_2$/feedstock molar ratio of less than 5.

13. Process according to any one of claims 5-11, which is carried out in the first stage at a temperature of 250 to 450 °C, a pressure of up to 50 bar, a space velocity of 0.5 to 10 g/g/h and a $H_2$/feedstock molar ratio of less than 5.

14. Process according to any one of claims 1-13, which is carried out in the second stage at a temperature of 50 to 400 °C and a pressure of up to 50 bar, a space velocity of 0.5 to 10 g/g/h and a $H_2$/feedstock molar ratio of less than 5.

15. Process according to any one of claims 1-14, wherein the hydrocarbonaceous feedstock substantially comprises linear paraffins having at least five to ten carbon atoms.

16. Process according to claim 15, wherein the hydrocarbonaceous feedstock substantially comprises linear paraffins having at least six to eight carbon atoms.

17. Process according to any one of claims 1-16, wherein the total effluent from the first reaction stage is passed to the second reaction stage.

**18.** Process according to any one of claims 1-16, wherein unconverted linear paraffins are separated from the effluent upstream the second reaction zone, and recycled to the first reaction zone.

**19.** Process according to any one of claims 1-17, wherein unconverted linear paraffins are separated from the effluent downstream the second reaction stage, and recycled to the first reaction stage.

**20.** Process according to any one of claims 1-18, wherein unconverted mono-branched paraffins are separated from the effluent downstream the second reaction stage, and recycled to the second reaction stage.

# FIG.1

# FIG.2

# FIG.3

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | US-A-4 232 181 (KIOVSKY ET AL)<br>----- | | C07C9/00<br>C07C5/27 |
| | | | **TECHNICAL FIELDS SEARCHED** (Int.Cl.6)<br><br>C07C |

The present search report has been drawn up for all claims

| Place of search | Date of completion of the search | Examiner |
|---|---|---|
| THE HAGUE | 26 January 1995 | Van Geyt, J |

EPO FORM 1503 03.82 (P04C01)